# EUROPEAN PATENT APPLICATION

(11) **EP 2 446 893 A1**
(43) Date of publication of application: **02.05.2012**
(21) Application number: 10189536.5
(22) Date of filing: 01.11.2010
(51) Int. Cl.: A61K 38/12, A61P 35/00

(54) **Use of nontoxic cyclic peptides to block transport via OATP1B1/ OATP1B3-related proteins, for the treatment of cancer cells expressing such transporters**

(71) Applicant: Bergen Teknologioverføring AS, 5006 Bergen (NO)
(72) Inventor: Herfindal, Lars, 5063 Bergen (NO); Doskeland, Stein Ove, 5221 Nesttun (NO)
(74) Representative: Gerstein, Hans Joachim

(57) **Abstract**

The present invention relates in a first aspect to cyclic peptides being stable and non-toxic for specifically antagonising OATP1B1 and/or OATP1 B3. That is, the present inventors identified OATP1B1 and/or OATP1B3 specific antagonists being storable and non-toxic. In particular, the present invention relates to the use of stable, non-toxic cyclic peptides whereby said cyclic peptides have a long shelf life as well as being stable under acidic conditions. Furthermore, the present invention relates to cyclic peptides wherein cyclisation is realised by amino linkage. Said cyclic peptides are particularly useful for the treatment of cancer. In addition, the cyclic peptides allow to design OATP1B1 or OATP1 B3 specific antagonising compounds useful for the treatment of cancer or modulate the transport to these channels, in particular modulation of the transport in liver cells for preventing drug metabolism or having anti-toxic activity against hepatotoxic compounds.

## Description

The present invention relates in a first aspect to cyclic peptides being stable and non-toxic for specifically antagonising OATP1B1 and/or OATP1B3. That is, the present inventors identified OATP1B1 and/or OATP1B3 specific antagonists being storable and non-toxic. In particular, the present invention relates to the use of stable, non-toxic cyclic peptides whereby said cyclic peptides have a long shelf life as well as being stable also under acidic conditions. Furthermore, the present invention relates to cyclic peptides wherein cyclisation is realised by amino linkage. Said cyclic peptides are particularly useful for the treatment of cancer. In addition, the cyclic peptides allow to design OATP1B1 or OATP1B3 specific antagonising compounds useful for the treatment of cancer or modulate the transport through these transporters also for other purposes, in particular modulation of the transport into liver cells for preventing drug or toxin entry.

### Background Art

The organic anion transporter proteins (OATP) have as major role to transport endogenous hormones and metabolites, as well as xenobiotics, including drugs into cells. The OATP transporters are highly conserved in evolution. This may explain why humans are susceptible to microbial toxins presumably designed to counteract primitive organisms. A striking example is microcystin (MC), which can cause acute, hemorrhagic liver damage whether ingested from contaminated water or dialysis fluid. Once MC has entered the hepatocyte it inhibits major Ser/Thr protein phosphatases, causing protein hyperphosphorylation, and reactive oxygen species (ROS) formation. The ensuing apoptosis is accompanied by polarized cell blebbing which may contribute to the disruption of the hepatic microvasculature and fatal bleeding.

The organic anion transporting polypeptides (humans: OATPs; rodents: Oatps) form a large gene superfamily of transport proteins expressed in multiple organs. Many of their members are multispecific transporters that mediate the uptake of numerous endo- and xeno-biotics. Human OATP1 B1 and OATP1B3 are expressed in the sinusoidal membrane of hepatocytes and are thought to be liver-specific under normal physiological conditions. They are responsible for the uptake of numerous drugs, including statins, endothelin receptor antagonists, anticancer drugs like methotrexate and paclitaxel, as well as the antibiotic rifampicin. Besides, specific substrates like prostaglandin E₂ for OATP1B1 and CCK-8 for OATP1 B3. OATP1B1 and OATP1 B3 have a number of common drug substrates including bosentan, fluvastatin, methotrexate, olmesartan, pitavastatin, rifampicin, rosuvastatin, and valsartan. Furthermore, these transporters can affect the bioavailability of drugs.

The importance of the OATP1B1 and 1 B3 proteins for drug transport is illustrated by the altered drug pharmacokinetics in patients with OATP1B1/1B3 mutations. The expression of OATP1B3 is normally limited to the liver, but it has recently been found to be expressed in cancers, notably colon carcinomas, where it facilitates the uptake of growth-promoting factors and confers apoptosis resistance. A non-toxic inhibitor of OATP1 B3 is therefore of interest not only to delay the uptake of potentially hepatotoxic natural compounds or drugs, but also to target OATP1 B3 in some aggressive cancers.

In WO 2007/138171 bioactive cyclic peptides are disclosed. Therein a nostocyclic peptide, Ncp-Ml is disclosed. Said cyclic peptide is derived from the cyanobacterium *nostoc sp.* Said nostocyclopeptide is characterized in having an imino bond for effecting cyclisation of the peptide. However, said imino bond renders the peptide unstable. In particular, the shelf life of the cyclic peptide is very low. Moreover, the cyclic peptide is not stable in solution, in particular in acidic solutions. Even at storage at -70ºC Ncp-M1 degrades, which is also the case when stored at room temperature, in particular in solutions having a pH below 7.

Recently it is described that Ncp-M1 is a cyanobacterial nostocyclopeptide representing a non-toxic anti-toxin against microcystins, Jokela J., et al., 2010 ChemBioChem, 11, 1594-1599.

Gui, C., et al, describes the development of a cell based high throughput assay to screen for inhibitors for organic anion transporting polypeptides 1 B1 and 1 B3, Current Chemicals Genomics, 2010, 4,1-8. It is identified therein that ursolic acid was the most selective OATP1B3 inhibitor being ten times more selective for OATP1 B3 compared to OATP1B1 while estropipate represents the most selective OATP1 B1 inhibitor (lC₅₀ = 0,06 µM vs. 19,3 µM of OATP1 B3).

Lee, W., et al., Cancer Res 2008; 68, 10315-10323 reports an overexpression of OATP1B3 in colon cancer. Said overexpression confers apoptotic resistance of colorectal cancer cells.

A first object of the present invention is to provide specific inhibitors of OATP1 B3 useful for the treatment of cancer. Another object of the present invention is to provide new non-toxic and stable cyclic peptides having OATP1B1 and/or OATP1 B3 antagonising activity. Said new compounds should allow to antagonise specifically OATP1B1 and/or OATP1 B3 activity and, thus, be useful for the treatment of cancer or to modulate transport through the OATP1B1 and/or OATP1B3 channels. In particular, said compounds shall modulate transport in liver cells to prevent drug metabolism or have anti toxic activity against hepatotoxic compounds.

### Summary of the invention

The present invention is based on the identification of novel cyclic peptides which are non-toxic and stable, thus, having a longer shelf life and being better able to withstand chemical conditions used to modify peptides (hemisynthesis). In addition, the new cyclic peptides and derivatives thereof, in particular their congeners, can be synthesized easily with common technology.

Moreover, the present invention is based on the finding that non-toxic, stable cyclic peptides having OATP1B1 and/or OATP1B3 antagonising activity are useful for the treatment of cancer, in particular, cyclic peptides having OATP1 B3 antagonising activity for the treatment of cancer, whereby said cancer cells express OATP1 B3. Moreover, the present invention relates to the use of said non-toxic stable cyclic peptides, in particular, of synthetic nostocyclopeptide derivatives having an amino bond (peptide bond), for the treatment of colon cancer, prostate cancer, and/or mammary cancer.

### Brief description of the drawings

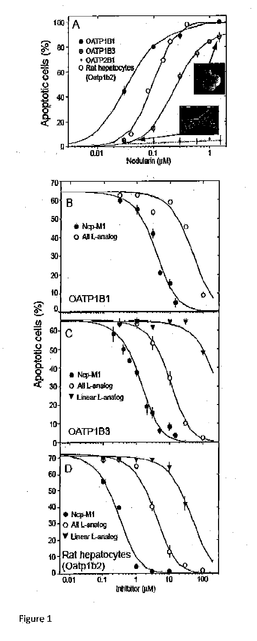

Figure 1: Ncp-M1 and synthetic Ncp-M1 analogs as inhibitors of nodularin (Nod)-induced apoptosis in OATP1B1 and 1B3-expressing HEK293 cells and in primary hepatocytes. A: HEK-293T cells co-transfected with GFP and OATP1B1, 1B3 or 2B1, and freshly isolated primary rat hepatocytes were assessed for apoptosis after 90 min incubation with various concentrations of Nod. The micrographs show OATP1B3-transfected HEK cells before (lower) and after (upper) exposure to Nod. B: OATP1B1-transfected HEK cells were preincubated (15 min) with Ncp-M1 or a cyclic Ncp-M1 peptide mimic, and thereafter with 0.1 µM Nod for 90 min before assessment of apoptosis. C: As for B except that the cells were transfected with OATP1 B3, and the concentration of Nod was 0.6 µM. D: Primary rat hepatocytes incubated with 70 nM Nod. The data are average values with SEM of 3-6 experiments.

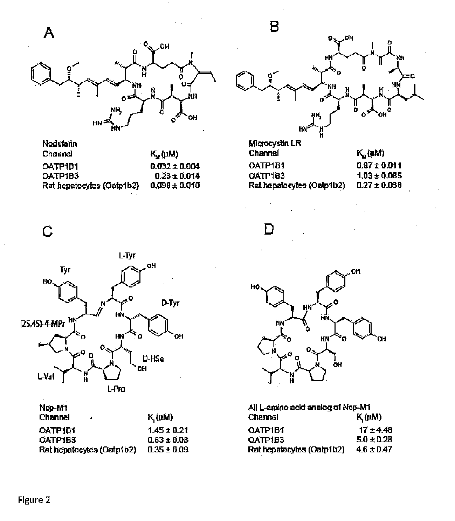

Figure 2: Structures of the cyclic peptides used, and their K_{M} or K, values with SEM for OATP1B1 and 1B3, and for rat hepatocytes (Oatb1 b2). The values were determined by non-linear regression from the data in Fig. 1, as described in the Experimental Section.

### Detailed description of the present invention

Unless otherwise specified, the terms used in the present invention, have the meaning communally used in the art to which this invention belongs.

In the following, the term "synthetic nostocyclopeptide derivatives" refers to cyclic peptides which have been synthesized chemically or biosynthetically through recombinant expression by appropriate methods known to the skilled person. Synthetic nostocyclopeptide derivatives have an amino bond for cyclisation, i.e. a peptide bond, in contrast to nostocyclopeptides, which are characterized in having an imino bond. Further, these derivatives are characterized in being composed of identical amino acids as the nostocyclopeptides, however, the amino acid may be in L- or D-form. Preferably, the amino acids are in the L-form.

The present inventors recognized that the Ncp-M1 nostocyclopeptide isolated from cyanobacteria is non-stable even when stored at -70°C and degrade in solution, in particular in acidic solutions. Thus, the present inventors aim in providing new cyclic peptides being stable even under moderately acidic conditions and having improved shelf life. Further, the cyclic peptides according to the present invention are characterized by having improved specificity and/or affinity either to OATP1 B3 or to OATP1 B1, respectively.

Further, the newly identified cyclic peptides as well as other non-toxic stable cyclic peptides according to the present invention are useful as OATP1 B1 and/or for OATP1 B3 antagonising medicaments, in particular, for the treatment of cancer, whereby said cancer cells express OATP1 B3.

In this connection, the term "non-toxic" means lacking morphologically adverse effects, growth inhibitory effects or gross perturbation effect of ser/thr protein phoshorylation in mammalian primary hepatocytes, which are the only normal mammalian cells known to express OATP1B1 or OATP1B3 like transporters and thereby being targeted by said peptides.

The term "stable" as used herein means that at least 90%, like at least 93%, e.g. 95%, 96%, 97%, 98% or 99% of the original compound as identified by HPLC-MS, and its anti-OATP1B1/3 activity can be recovered after storage for 6 months under standard conditions at -40°C, or after at least 12 h in physiological solution, isotonic (0.9 %) NaCl, or standard cell culture medium, Dulbecco DMEM at 37 °C, or for 2h in moderately acidic buffers, namely 0.05% tri-fluoro acetic acid or 0.1 % acetic acid. The term "peptide" refers to a molecule formed from the linking, in a defined order, of various amino acid residues, including derivatives. The peptide can be of any length, at least two, usually at least 3, 4, 5 or 6 to 7, often about 7, generally 7 to 8, normally about 9, 10, 11, 12, 13 or 14 or more amino acid residues. The peptide or polypeptide can be composed of amino acids joined to each other by peptide bonds or modified peptide bonds, i.e., peptide stereoisomers, and may contain amino acids other than the gene-encoded amino acids.

"Peptides" can include coded and non-coded amino acids, chemically or biochemically modified, for example post-translationally modified such as glycosylated or derivatized amino acids, polymeric polypeptides and polypeptides having modified peptide backbones. The "peptides" or "polypeptides" may be modified by either natural process, such as posttranslational processing, or by chemical modification techniques which are well known in the art. Such modifications are well described in basic texts and in more detailed monographs, as well as in a voluminous research literature. Modifications can occur anywhere in a peptide or polypeptide, including the peptide backbone, the amino acid side-chains and the amino or carboxyl termini. It will be appreciated that the same type of modification may be present in the same or varying degrees at several sites in a given peptide or polypeptide. Also, a given peptide or polypeptide may contain many types of modifications. Peptides or polypeptides may be branched, for example, as a result of ubiquitination, and they may be cyclic, with or without branching. Cyclic, branched, and branched cyclic polypeptides may result from posttranslation natural process or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, Proteins - structure and molecular properties, 2nd Ed., T. E. Creighton, W. H. Freeman and Company, New York (1993); Postranslational covalent modification of proteins, B. C. Johnson, Ed., Academic Press, New York, pgs. 1-12 (1983); Seifter et al, Meth Enzymol 182:626- 646 (1990); Rattan et al., Ann NY Acad Sci 663:48-62 (1992)).

The term "cyclic peptide" or "cyclopeptides" or cyclic protein refers to a peptide or polypeptide whose amino and carboxy termini are themselves linked together with a peptide bond (amino bond) or modified peptide bond, forming a circular chain unless otherwise identified.

The newly identified cyclic peptides are characterized in being non-toxic and stable. Consequently, said novel cyclic peptides can replace toxic and less specific hepatic transport inhibitors. Moreover, they provide also a novel, exopeptidase-resistant scaffold for the synthesis of novel modulators of OATP1 B1 and the potential drug target OATP1 B3.

In a preferred embodiment of the present invention, the cyclic peptide has the sequence Seq. ID No.1 Tyr₁-Tyr₂-Ser-Pro-Val-Pro-Tyr₇ (SEQ ID No. 1 ) having an amino bond between Tyr₁ and Tyr₇, or derivatives thereof, and solvates thereof as well as pharmaceutically acceptable salts thereof.

In a preferred embodiment, the cyclic peptide is an all L-amino acid cyclic peptide. Particularly preferred, the cyclic peptide according to the present invention is a peptide of formula I

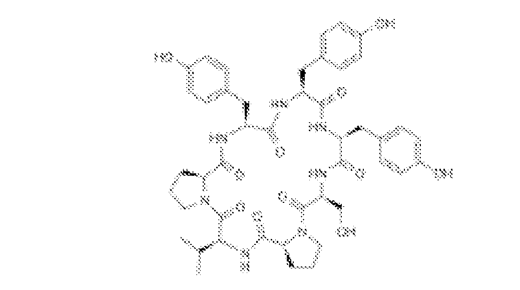

These newly described cyclic peptides according to the present invention represent useful prophylactic or therapeutic active substances. Accordingly, the present invention relates to pharmaceutical compositions containing the novel cyclic peptides according to the present invention.

Preferably, the pharmaceutical composition is provided in a form suitable for oral, topical, transdermal, injection, buccal, transmucosal, pulmonary or inhalation administration.

The non-toxic, stable cyclic peptide according to the present invention is useful for the manufacture of an OATP1B1 and/or OATP1 B3 antagonising medicament. Said non-toxic stable cyclic peptide is preferably a synthetic nostocyclopeptide derivative. Particularly preferred, said non-toxic stable cyclic peptide according to the present invention is useful for the treatment of cancer, whereby said cancer cells express OATP1 B3. Particularly preferred, the cancer is selected from colon cancer, prostate cancer and/or mammary cancer.

Further, the cyclic peptides according to the present invention are useful as antagonists of OATP1B1 and/or OATP1B3, thus, allowing treatment of a cancer or modulation of transport through these channels, in particular, modulating transport deliver cells for preventing drug metabolism or have an antitoxic activity against hepatotoxic compounds.

As shown in the examples below, the non-toxic stable cyclic peptides according to the present invention allow to block the entry of toxic compounds, like nodularin and microcystin as representative of hepatotoxic compounds. Hence, it is possible to inhibit uptake of a hepatotoxic compound as well as uptake of prodrugs or drugs, accordingly.

That is, the compound according to the present invention allows delaying (inhibiting) the uptake of hepatotoxic compounds, prodrugs or drugs. Thus, it is also possible to use the compounds according to the present invention to treat individuals suffering from diseases, disorders or conditions resulting from extensive administration of prodrugs or drugs, accordingly.

The present invention also relate to pharmaceutical composition comprising a cyclic peptide according to the present invention, in particular cyclic peptides of Seq. ID1 or derivatives thereof. In a preferred embodiment, the peptide is a peptide of a formula I. The present invention is related to the use of peptide of the invention for the manufacture of medicine for the treatment against hepatotoxic as well as for the treatment of cancer.

There is a great need for novel peptides in compounds effective for protecting subjects against the toxic influence of hepatotoxins, like cyanobacterial toxins, especially those which exist in increasing amounts in the algal blooms around the world. The present invention accordingly relates to a novel peptide useful in protecting hepatocytes against lethal influence of cyanobacterial toxins, like microcystin and nodularin.

In another aspect, the peptides according to the present invention can be useful for screening new molecules having OATP1 B1 and/or OATP1 B3 antagonising activity. Further, it is possible to use the cyclic peptides described herein for the design and synthesize of non-toxic inhibitors and further improve affinity for this report.

The present invention will be described further with the following examples, which are provided by a way of illustration or not intended to limiting the present invention.

### Experimental section

### Materials

Nostocyclopeptide-Ml (Ncp-M1 ) was purified to homogeneity from a culture of the *Nostoc cyanobacteria strain* XSPORG 13A, as described previously, Jokela J., et al., 2010 ChemBioChem, 11, 1594-1599. Ncp-M1 was kept at -80°C until dissolved in DMSO before use.

The synthetic all L-amino acid cyclic and linear peptide analogs of Ncp-M1 (Tyr-Tyr-Ser-Pro-Val-Pro-Tyr) (Seq. ID. No. 1) were from Peptides International (Louisville, KY). Microcystin YR (MC-YR) was labelled with **[¹²⁵l]** and purified by reversed phase HPLC. Nodularin was labelled with ³H by reduction with sodium boro[³H]hydride. C-glycocholic acid was from Amersham Biosciences, (Little Chalfont, UK), and bromosulfophthalein (BsP) and cholic acid from Sigma-Aldrich (St. Louis, MO).

### Cell experiments

Rat hepatocytes were isolated by *in vitro* collagenase perfusion, Mellgren G, et al., Journal of Cellular Physiology 1995;163:232-240. Experimental conditions were as described in Herfindal L, et al., J Med Chem 2009;52:5758-5762, except for assay of NaN₃ and general cytotoxicity, where the hepatocytes were seeded in collage-coated 24-well culture dishes. Microinjection of hepatocytes was as described previously, Fladmark KE, et al., Hepatology 1997;25:847-855. The hepatocyte death (by apoptosis or necrosis) was evaluated by microscopy. Long-term hepatocyte viability was assessed also by the WST-1 colorimetric assay of mitochondrial dehydrogenase activity (Roche Applied Sciences, Mannheim, Germany).

Human embryonic kidney cells (HEK 293T) were cultured in DMEM supplemented with 10% heat-inactivated fetal bovine serum in 6-well cell culture plates. Enforced expression of GFP alone or with OATP1B3, OATP1B1 or OATP2B1 was by calcium phosphate transfection of 70 % confluent HEK 293T cells for 6 h. After 20 h the cells were detached by gentle trypsin treatment and seeded in 48-well tissue culture plates (30 000 cells/well). After 24 h 80 - 90 % of the cells were GFP positive by fluorescence microscopy, and they were exposed to MC or Nod with or without anti-toxin. The effect of MC and Nod was assessed by microscopy of the GFP expressing cells. Vectors for OATP1B1, 1 B3 and 2B1 were a gift from Dietrich Keppler, German Cancer Research Center, Division of Tumor Biochemistry, Heidelberg, Germany.

### Determination of hepatocyte uptake of radiolabeled MC-YR, nodularin and glycocholic acid

Freshly prepared primary rat hepatocytes in suspension (800 000 cells/ml) were incubated in buffer with Ncp-M1, BSP or vehicle for 15 minutes (37°C in humidified atmosphere with 6 % C0₂) under gyratory shaking. Radiolabeled ['251]MC-YR or [³H]Nodularin was added and the cells incubated for another 5 minutes before rapid separation from the medium by centrifugation through a layer of a mixture of dibutyl and dinonyl phthalate. The cell pellet was lysed in 1 ml of 2 % SDS for 1 hour and the radioactivity determined by scintillation counting (TriCarb 3100TR, Packard Bioscienses, CA). The accumulation of [¹⁴C]glycodeoxycholate was determined similarly, except that the cells were separated from the medium by gentle filtration (0.45 pm HA filters, Millipore Billerica, MA). The cell pellet was washed twice with 2 ml of incubation medium (37°C), and the filters processed for scintillation counting.

### Determination of CaM-KII activity and protein phosphorylation

The in vitro CaM-KII activity was assayed at 30°C in the absence and presence of 20 µM Ncp-M1 or 10 µM KN-93 using syntide-2 (PLARTLSVAGLPGKK) (Seq. ID. No. 2) as substrate. The basic incubation mixture contained 15 mM Hepes pH 7.40 with 10 mM MgCl₂, 0.5 mM [^{32p}] -_{Y}-ATP (0.1 pCi/50 pl), 1 mM Ca²⁺, 10 µg ml⁻¹ calmodulin, and 1.5 µg ml-¹ CaM-Kll.

The intact cell protein phosphorylation was determined in freshly isolated hepatocytes in suspension preincubated with ³² Pᵢ and 15 µM Ncp-M1 or vehicle for 15 min before the addition of 200 nM nodularin. After another 20 min incubation the cells were precipitated with 7 % trichloroacetic acid (TCA), the pellet washed with 5 % TCA followed by extraction with ether, and solubilized in standard Tris buffer with SDS before separation by PAGE in linear gradient polyacrylamide gel (7-15%). Phosphoproteins were visualized by exposing the dried gel to Kodak bio max film.

To estimate the effect of Ncp-M1 on in vitro phosphorylation of endogenous liver proteins, a cytosol fraction was prepared by ultracentrifugation (100 000 x g, 1 h) of rat liver homogenized in 50 mM Hepes pH 7.2 containing 120 mM KC1, 5 mM EDTA, 3 mM EGTA, 2 mM dithioerythritol, 10 mM benzamidine, 50 µg ml⁻¹ soya bean trypsin inhibitor, 50 µg ml-¹ aprotinin, 7 µg ml-¹ chymostatin, 6 µg ml-¹ antipain, 48 µg ml-¹ leupeptin, and 14 pg ml-' pepstatin. The phosphorylation of cytosol proteins was studied in the presence or absence of the following: 1 µg ml-¹ CaM-Kll (Upstate, USA), 1 µM Nod, 20 µM Ncp-M1, 10 µM KN-93. All samples were incubated at 25°C in 50 mM Hepes pH 7.2 with 20 mM protein kinase A inhibitor, 5 mM McCl₂, 50 µM [³²P] γ-ATP (0.2 µCi/50 µl), 50 µg ml-¹ SBTI, 1 mM Ca ²⁺, 10 µg ml-¹ calmodulin and 5 mg ml-¹ cytosol protein. The phosphate incorporation increased rectilinearly with time for about 45s, and the assay duration was 30 s.

### Statistical analysis and estimation of K_{M} and K, values

The data for accumulated radiolabeled nodularin and glycocholic acid into hepatocytes in the absence and presence of uptake inhibitor were analyzed for significance by Wilcoxon Signed-Rank and students t-tests, using the SPSS statistical software (Chigaco, IL). Estimations of the apparent K_{M} and Kₗ, values for Mc/Nod uptake as deduced from HEK 293 cell apoptosis were done using SigmaPlot software (Systat Software inc. San Jose, CA). A competitive Michaelis-Menten equation with Hill-constant was used to model the plots shown in Fig. 1, and the *K_{M} and Kₗ values* were determined by non-linear regression analysis.

### Characterization of the Ncp-M1 inhibition of the OATPIB/1B3 transporters

The MC uptake in rodent hepatocytes is mainly mediated by Oatp1b2, whose two human orthologs, OATP1B1 and OATP1B3 allow MC entry when expressed in frog oocytes or HEK 293 cells. It has been identified that HEK 293T cells with enforced expression of OATP1B1 or OATP1 B3 became about as sensitive as rat hepatocytes towards nodularin-induced apoptosis (Fig. 1A). Cells expressing the BSP-sensitive hepatocyte sinusoidal membrane transporter OATP2B1 (Fig. 1 B) or OATP1 B3 (Fig. 1 C) could be completely counteracted by Ncp-M1. This provides direct evidence that Ncp-M1 inhibits transport through the two human MC-carrying channels.

Next a synthetic cyclic Ncp-M1 derivative of formula l according to the present invention in which the unusual Ncp-M1 amino acids (2S,4S)-4-methyl-proline, D-homoserine, and D-tyrosine were replaced by L-proline, L-serine, and L-tyrosine, and the imino-bond replaced by an ordinary peptide bond (see Fig. 2 for structures) has been tested. It inhibited completely the uptake of nodularin through OATP1B1/B3 and the rat hepatocyte channel Oatp1b2, albeit with lower potency than Ncp-M1 (Fig. 1B-D). Like Ncp-M1, the derivative according to the present invention was non-toxic for both primary rat hepatocytes and for HEK 293 cells with and without OATP1B1/B3 overexpression (not shown). A linear version of the same peptide was considerably less potent (Fig. 1 C, D), indicating that the cyclic structure is important for high-affinity channel recognition.

The apparent K_{M} for MC and Nod and the K, of Ncp-M1 and the cyclic peptide derivative according to the present invention are listed in Fig. 2. Ncp-M1 and microcystin-LR had similar affinity for OATP1 B1 (Kₗ =1.5pM, K_{M} =1 pM), OATP1 B3 (Kᵢ=10.63pM, K_{M}=1 pM) and the rat hepatocyte (Oatp1 b2) channel ((Kᵢ =0.35pM, K_{M} =0.27pM). This does not mean that such cyclic peptides cannot discriminate between the channels, since nodularin had a particularly high affinity for the OATP1 B1 channel (K_{M} =32nM).

Hence a stable, nontoxic, and inexpensive Ncp-M1 derivative, the synthetic nostocyclopeptide derivatives according to the present invention can inhibit transport through both OATP1 B1/B3 and their rodent ortholog Oatp1 b2 and therefore represents a novel useful tool to block transport through these channels. It can also serve as a scaffold for the production of second generation channel modulators.

The cyclic peptide antitoxin Ncp-M1 protects hepatocytes against the death-inducing effect of microcystins and nodularin. Ncp-M1 blocks the hepatocyte uptake of microcystin and nodularin as well as the uptake of nodularin and MC into HEK293 cells with enforced expression of either of the two human hepatocyte microcystin transporters OATP1 B1 and OATP1 B3. Ncp-M1 did not interfere with intracellular steps of microcystin action, such as protein phosphatase inhibition, CaM-KII activation, ROS production, or actin rearrangement. The apparent intracellular inertness of Ncp-M1 distinguishes it from other OATP1 B1/1 B3 inhibitors. Common inhibitors are usually biologically active endogenous substances (e.g. steroid hormones, bilirubin or bile salts), toxins (e.g. phalloidin), biologically active xenobiotics (e.g. ursolic acid), or drugs (e.g. fluvastatin and methotrexate.

Ncp-M1 as well as the cyclic peptide of Seq. ID. No. 1 showed no apparent inherent toxicity, as evidenced by the normal morphology, growth and mitochondrial dehydrogenase activity of hepatocytes incubated with it for 96 hours. The much used non-specific hepatocyte channel blocker BSP was under similar conditions highly toxic for hepatocytes. Ncp-M1 as well as the cyclic peptide of Seq. ID. No. 1 has also other advantages like high solubility in physiological saline and exopeptidase resistance due to its cyclic peptide nature.

Thus, Ncp-M1 as well as the cyclic peptide of Seq. ID. No. 1 represent novel inhibitors of the human hepatocyte OATP1 B1/1 B3 and rodent hepatocyte Oatp1b2. The synthetic all L-amino acid cyclic peptide Ncp-M1 derivative according to the present invention is apparently as potent to inhibit OATP1B3 transport as the best inhibitors recently found by screening the 1100 drugs of the Prestwick library, Gui, C., et al, Current Chemicals Genomics, 2010, 4,1-8. In addition it has preference for inhibition of OATP1 B3. Its synthetic all L-amino analog has even higher OATP1 B3 preference (Fig. 2D), suggesting that these peptides can be useful scaffolds for the synthesis of novel non-toxic inhibitors with further improved affinity for this transporter. A selective inhibitor is needed to discriminate between the transport through the related OATP1B1 and OATP1B3 in the liver itself. It has also the potential to spare the uptake of many endogenous compounds taken up redundantly by the liver through OATP1B1 and OATP1 B3, while depriving aggressive tumors of the growth factors apparently obtained by ectopic expression of the OATP1 B3 transporter.

## Claims

1. The use of a non-toxic, stable cyclic peptide, in particular, of synthetic nostocyclopeptide derivatives having an amino bond for cyclisation, for the manufacture of an OATP1 B1 and/or OATP1 B3 antagonising medicament.

2. The use according to claim 1, wherein said OATP1 B1 and/or OATP1 B3 antagonising medicament is intended to be used for the treatment of cancer, in particular, where cancer is mediated by or resulting from OATP1 B1 and/or OATP1 B3 activity.

3. The use according to any one of the preceding claims for the treatment of cancer, whereby said cancer cells express OATP1 B3.

4. The use according to claim 3 for the treatment of colon cancer, prostate cancer, and/or mammary cancer.

5. A cyclic peptide of the sequence Tyr₁-Tyr₂-Ser-Pro-Val-Pro-Tyr₇ (SEQ ID No. 1) having an amino bond between Tyr₁ and Tyr₇ or derivatives thereof, and solvates thereof, and pharmaceutically acceptable salts thereof.

6. A cyclic peptide according to claim 5, which is an all L-amino acid cyclic peptide.

7. The cyclic peptide according to claim 5 or 6 **characterized in that** it has the formula I

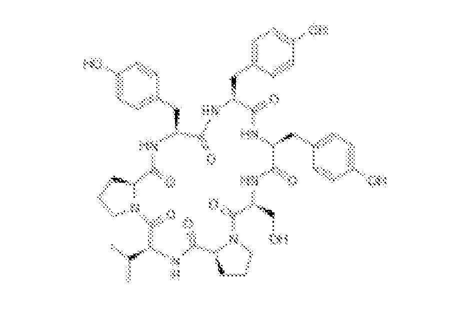

8. The cyclic peptide according to any one of claims 5 to 7 for use as prophylactically or therapeutically active substances.

9. The cyclic peptide according to any one of claims 5 to 8 having OATP1 B1 and/or OATP1 B3 antagonising activity and being useful for the treatment of cancer, or where cancer is mediated by or resulting from OATP1 B1 and/or OATP1 B3 activity; or modulate transport through said channels, in particular, modulate transport in liver cells for preventing drug metabolism or having antitoxic activity against hepatotoxic compounds.

10. Pharmaceutical composition containing a peptide according to any one of claims 5 to 8 and a pharmaceutically acceptable carrier.

11. Pharmaceutical composition according to claim 10 in a form suitable for oral, topical, transdermal, injection, buccal, transmucosal, pulmonary or inhalation administration

12. The use of the cyclic peptides according to any one of claims 5 to 8 for delaying or inhibiting uptake of hepatotoxic compounds, prodrugs or drugs.

13. The use according to any one of claims 5 to 8 for the treatment of cancer, in particular colon cancer, prostate cancer and/or mammary cancer.

14. The use of the peptide according to any one of claims 5 to 8 for screening methods.

15. The use of a cyclic peptide according to any one of claims 5 to 8 for the design of OATP1 B1 and/or OATP1 B3 antagonising molecules.


SEQUENCE LISTING

<110>  Bergen Teknologioverforing AS

<120>  Use of nontoxic cyclic peptides to block transport via OATP1B1/
       OATP1B3-related proteins, for the treatment of cancer cells
       expressing such transporters

<130>  4200-012-EP-1

<160>  2

<170>  PatentIn version 3.3

<210>  1
<211>  7
<212>  PRT
<213>  artificial

<220>
<223>  cyclic peptide

<400>  1

Tyr Tyr Ser Pro Val Pro Tyr
1               5


<210>  2
<211>  15
<212>  PRT
<213>  artificial

<220>
<223>  oligopeptide syntide-2

<400>  2

Pro Leu Ala Arg Thr Leu Ser Val Ala Gly Leu Pro Gly Lys Lys
1               5                   10                  15